# EUROPÄISCHE PATENTSCHRIFT

(11) **EP 0 678 498 B1**
(45) Veröffentlichungstag und Bekanntmachung des Hinweises auf die Patenterteilung: **07.10.1998**
(21) Anmeldenummer: 95105397.4
(22) Anmeldetag: 10.04.1995
(51) Int. Cl.: C07C 68/08, C07D 317/38

(54) **Verfahren zur Reinigung von Ethylenglykolcarbonat (EGC) durch Adsorption an Aktivkohle**
Process for the purification of ethyleneglycol carbonate through adsorption on active carbon
Procédé de purification du carbonate d'éthylène glycol par adsorption sur du charbon actif

(30) Priorität: 22.04.1994 DE 4414121
(43) Veröffentlichungstag der Anmeldung: 25.10.1995
(73) Patentinhaber: BAYER AG, 51368 Leverkusen (DE)
(72) Erfinder: Mendoza-Frohn, Christine, Dr., D-40699 Erkrath (DE); Wagner, Paul, Dr., D-40597 Düsseldorf (DE); Buysch, Hans-Josef, Dr., D-47809 Krefeld (DE)

(56) Entgegenhaltungen:
- PATENT ABSTRACTS OF JAPAN vol. 011 no. 047 (C-403) ,13.Februar 1987 & JP-A-61 210057 (TOKUYAMA SODA CO LTD) 18.September 1986,
- PATENT ABSTRACTS OF JAPAN vol. 004 no. 098 (C-018) ,15.Juli 1980 & JP-A-55 059144 (TOKUYAMA SODA CO LTD) 2.Mai 1980,

## Beschreibung

Die vorliegende Erfindung betrifft ein Verfahren zur Reinigung von Ethylenglykolcarbonat (EGC), welches Verunreinigungen aus dem Herstellungs- bzw. Aufarbeitungsverfahren enthält, durch Behandlung mit Aktivkohle.

Das Verfahren ermöglicht beispielsweise die Reinigung eines EGC-Stromes, der aus dem Reaktionskreislauf zur Herstellung von EGC zwecks Vermeidung eines Nebenproduktakkumulation ausgeschleust wird.

Das Verfahren erlaubt die praktisch vollständige Rückführbarkeit dieses gereinigten EGC-Seitenstromes in den EGC-Herstellungs- und Aufarbeitungskreislauf und trägt dadurch zu einer materialsparenden Fahrweise bei der Herstellung von EGC bei.

Es ist bekannt, daß EGC auf verschiedenen Wegen hergestellt werden kann, beispielsweise aus Ethylenglykol und Phosgen (DE-AS 1 226 117), aus Ethylenoxid bzw. Ethylenchlorhydrin und Kohlendioxid (Chem. Ing. Techn. 43 (1971), 903 ff.; Fette, Seifen, Anstrichmittel 73 (1971), 396 ff.; DE-OS 2 855 232, entsprechend US 4.314.945; Ind. Eng. Chem. 50 (1958), 767-770; DE-OS 4.141.189, DE 4.138.438; DE 4.210.943) sowie aus Ethylen, Sauerstoff und Kohlendioxid (US 3.025.305). Hierbei fällt das EGC in der Regel in einer durch die verschiedenen Stoffe aus dem Herstellungsprozeß und Nebenkomponenten verunreinigten Form an.

Bei dem von Ethylenoxid und Kohlendioxid ausgehenden Verfahren enthält das Rohprodukt weiterhin den gelösten Katalysator, beispielsweise quartäre Ammoniumverbindungen, Alkalihalogenide oder Alkalihalogenide in Kombination mit Zinkhalogeniden. Dieser muß vor einer Feinreinigung, z.B. durch fraktionierte Destillation aus dem Rohprodukt abgetrennt werden. So werden beispielsweise bei dem in Chem. Ing. Techn. und Fette, Seifen, Anstrichmittel (loc. cit.) beschriebenen Verfahren vor der Feinreinigung zwei Dünnschichtdestillationen zur Abtrennung des Katalysators durchgeführt. Dieser abgetrennte Katalysator kann dann größtenteils wieder in der Herstellungsreaktion zum EGC zurückgeführt werden. Bei dieser Dünnschichtdestillation wird das EGC teilweise zersetzt, auch wenn milde Bedingungen (maximal 50 mbar) eingehalten werden. Die Zersetzungsprodukte sammeln sich je nach Flüchtigkeit im Destillat bzw. im Sumpf der Destillation. Die Gefahr der Zersetzung des EGC besteht insbesondere dadurch, daß der Katalysator durch das Abdestillieren des reinen EGC im Destillationssumpf aufkonzentriert wird und der Grad der Zersetzung des EGC stark von der Konzentration des in ihm gelösten Katalysators abhängt. So darf bei einer Eingangskonzentration von 0,25 bis 0,5 Gew.-% an Katalysator nicht mehr als 90 bis 95 % des EGC abdestilliert werden. Darüber hinaus kann der Katalysator selber bei diesem Destillationsvorgang zersetzt werden. Will man also den Destillationssumpf, der eine konzentrierte Katalysatorlösung darstellt, in die Herstellungsreaktion zurückzuführen, muß ein Anteil des Katalysators verworfen und durch einen neuen Katalysator ersetzt werden. So wird beispielsweise ein Ersatz von 30 % der Katalysatorlösung empfohlen. Das Ersetzen der Katalysatorlösung erfolgt durch Ausschleusen eines Anteils der Katalysatorlösung aus dem EGC-Herstellungs und Aufarbeitungskreislauf und gleichzeitiges Einspeisen der entsprechenden Menge an frischem Katalysator (Ind. Eng. Chem., loc. cit; DE 4.141.189).

Durch die Ausschleusung vermeidet man außerdem eine Akkumulation an Verunreinigungen, z.B. höhersiedenen Nebenkomponenten, die sich während der Reaktion und der Aufarbeitung bilden können.

Der Ausschleusestrom kann, je nach den Bedingungen der EGC-Herstellung und -Aufarbeitung, bis zu ≥ 90 % aus EGC bestehen und bedeutet daher eine deutliche Einbuße an Ausbeute, wenn er verworfen werden muß.

Dies gilt insbesondere dann, wenn Herstellung und Aufarbeitung schonend durchgeführt werden, beispielsweise durch adiabate Herstellung des EGC wie in DE 4.141.189 unter Verwendung niedriger Drücke und geringer Abtrennraten bei der Vakuumdestillation. Bei solcher schonenden Herstellung und Aufarbeitung kann der Ausschleusestrom sogar zu ca. 99 % aus EGC bestehen.

Aus JP-A 55/59 144 (1980) und JP-A 61/210 057 (1986) ist bekannt, Diallylcarbonat oder Dimethallylcarbonat durch Behandlung mit Aktivkohle von färbenden Komponenten zu befreien. Gemaß JP '144 kann der Behandlung mit Aktivkohle eine Behandlung mit Oxidationsmitteln vorausgehen und die Behandlung in Gegenwart von Mineralsäure durchgeführt werden. Solche Carbonate mit ungesättigten Gruppen werden beispielsweise als Startmaterial für optische Linsen eingesetzt.

Es war daher wünschenswert, ein Reinigungsverfahren für durch höhersiedende Nebenkomponenten verunreinigtes und evtl. mit Katalysator aus dem EGC-Herstellungsprozeß beladenes EGC zu haben, das zur möglichst vollständigen Entfernung der Nebenkomponenten, aber nur zu geringen Katalysatorverlusten führt. Das Reinigungsverfahren sollte darüber hinaus selbst keine Belastung für EGC und Katalysator darstellen und energiesparend sein.

Es wurde nun überraschenderweise gefunden, daß bei der Behandlung von EGC-Rohprodukten bzw. von mit höhersiedenden Nebenkomponenten aus dem EGC-Herstellungs- bzw. Aufarbeitungsprozeß verunreinigten EGC-Katalysatorlösungen mit Aktivkohle die Nebenkomponenten praktisch vollständig aus dem EGC entfernt werden, während der Katalysator nur zu geringen Teilen an der Aktivkohle adsorbiert wird. Auf diese Weise kann die Verbrennung des zu 90 bis ca. 99 % aus EGC bestehenden Ausschleusestromes vermieden und der gereinigte Strom in den Herstellungsprozeß zurückgeführt werden. Der an der Aktivkohle adsorbierte geringe Anteil des Katalysators muß bei der Rückführung des gereinigten EGC in den Reaktionskreislauf durch frischen Katalysator ersetzt werden. Dieser Ersatz eines geringen Anteils an adsorbiertem Katalysator kann sogar erwünscht sein, wenn hierdurch eine gegebenenfalls erfolgte Katalysatordesaktivierung während der Reaktion und Aufarbeitung ausgeglichen wird.

Eine weitere vorteilhafte Wirkung der erfindungsgemäßen Reinigung von EGC durch Adsorption ist die, daß der Reinigungsschritt selbst keine zusätzliche Belastung für EGC und Katalysator darstellt.

Gegenstand der Erfindung ist demnach ein kontinuierlich oder diskontinuierliches Verfahren zur Reinigung von Ethylenglykolcarbonat (EGC), welches Verunreinigungen aus dem Herstellungs- bzw. Aufarbeitungsverfahren des EGC enthält, das dadurch gekennzeichnet ist, daß das verunreinigte EGC bei 40-250°C, bevorzugt 40-200°C, besonders bevorzugt 40-160°C und bei 1-200 bar, bevorzugt 1-100 bar, besonders bevorzugt 1-30 bar einer Behandlung mit Aktivkohle mit einer BET-Oberfläche von 200-3000 m²/g, bevorzugt 300-2000 m²/g, besonders bevorzugt 500-1500 m²/g unterworfen wird.

Als erfindungsgemäß einzusetzendes verunreinigtes EGC kommt sowohl ein vorgereinigtes EGC in Frage, das einer weiteren Feinreinigungsstufe unterzogen werden soll, als auch ein rohes Reaktionsgemisch, das direkt aus dem Herstellungsprozeß des EGC stammt. Wegen der oben beschriebenen praktisch vollständigen Entfernung der höhersiedenden Nebenkomponenten bei gleichzeitig nur geringer Adsorption des Katalysators eignet sich die Reinigungsmethode in bevorzugter Weise für die Aufarbeitung des Ausschleusestromes, der zur Vermeidung einer Nebenproduktakkumulation aus dem Reaktions- und Aufarbeitungskreislauf herausgeführt wird und der dennoch zum überwiegenden Anteil aus EGC besteht.

In weiterhin vorteilhafter Weise kann das Reinigungsverfahren dann zur gleichzeitigen Entfernung eines Anteils an Katalysator zwecks Erneuerung von desaktiviertem Katalysator dienen.

Dem Fachmann ist die Akkumulationsrate an Nebenkomponenten im Kreislauf aus Reaktion und Aufarbeitung und damit der notwendige Ausschleusestrom zur Vermeidung einer Nebenkomponentenakkumulation bekannt. Durch die Reinigung mit Aktivkohle kann in Abhängigkeit von den reaktionstechnischen Parametern (wie z.B. Verweilzeit) in einer dem Fachmann bekannten Weise eine praktisch vollständige Entfernung der Nebenkomponenten aus dem Ausschleusestrom stattfinden. Die in weitaus geringerem Maße stattfindende Adsorption des Katalysators an der Aktivkohle hängt ebenfalls von den gewählten Parametern der Adsorption ab. Zum Zweck des Austausches an desaktiviertem Katalysator ist jedoch eine im Einzelfall leicht zu bestimmende Menge an Katalysator aus dem Kreislauf zu entfernen.

Dementsprechend läßt sich abwägen, ob der Ausschleusestrom vollständig oder nur teilweise über die Aktivkohlereinigung geleitet und ob bei dieser Reinigung durch geeignete Wahl der Parameter eine vollständige Entfernung der Nebenkomponenten herbeigeführt wird.

In weiterhin bevorzugter Weise wird man den Ausschleusestrom aus dem Rückstand der Aufarbeitung entnehmen, da hier die Konzentration an Nebenkomponenten am höchsten und eine Reinigung des Ausschleusestromes am effektivsten ist. Bei der Aufarbeitung durch Vakkumdestillation entspricht der Rückstand dem Destillationssumpf. Es sind jedoch auch andere Aufarbeitungsmethoden für das EGC denkbar, und die Reinigung des Ausschleusestromes ist dann ebenfalls sinnvoll und durchführbar. Beispielsweise kann gereinigtes EGC aus einem verunreinigtes EGC, welches Verunreinigungen aus der Gruppe von Ausgangsstoffen, Nebenprodukten und/oder Katalysatoren des Herstellungsverfahrens enthält, durch fraktionierende Schmelzkristallisation erhalten werden Hierbei stellt das gebildete Kristallisat das gereinigte EGC dar, die Restschmelze, in der die Verunreinigungen gelöst sind, den zurückführenden Rückstand.

Es sind ebenfalls Kombinationen von Aufarbeitungsmethoden denkbar, beispielsweise eine Schmelzkristallisation und eine anschließende Vakuumdestillation des Kristallisats. In diesem Fall können die Schmelze aus der Kristallisation und der Destillationssumpf in den Herstellungsprozeß zurückgeführt werden und stellen innerhalb des Prozesses den geeigneten Ort für die Entnahme des Ausschleusestromes dar.

In bevorzugter Weise wird der durch Adsorption an Aktivkohle gereinigte EGC-Strom in den Herstellungskreislauf zurückgeführt. Das erfindungsgemäße Verfahren zur Reinigung von EGC durch Adsorption an Aktivkohle kann sowohl unter absatzweiser als auch unter kontinuierlicher Kontaktierung der Aktivkohle mit dem zu reinigenden EGC durchgeführt werden; bevorzugt wird es unter kontinuierlicher Kontaktierung durchgeführt.

Für die erfindungsgemäße Reinigung von EGC durch Adsorption an Aktivkohle können Adsorptionsverfahren eingesetzt werden, wie sie beispielsweise in Ullmann's Enzyklopädie der technischen Chemie, 4. Auflage, Vol. 2, S. 600-619 und in der dort zitierten Literatur beschrieben und dem Fachmann bekannt sind. Hierbei handelt es sich beispielsweise um Festbettverfahren, bei denen die Aktivkohle fest angeordnet und durch das zu reinigende Edukt in geflutetem Zustand bzw. in der Kieselphase abwärts oder aber von unten nach oben durchströmt wird.

Von der Bauweise des Festbettadsorber her unterscheidet man z.B. Horizontal-oder Vertikaladsorber und einfache Adsorptionsbecken. Andere Adsorptionsverfahren arbeiten statt dessen mit bewegten Adsorptionsmittel, beispielsweise in der Wanderschicht oder in der Wirbelschicht. Bei Einsatz von pulverförmigen Aktivkohlen unterscheidet man weiterhin Einrührverfahren, bei denen das Adsorptionsmittel mit dem zu reinigenden Edukt in einem Rührbehälter verrührt und anschließend in einer Filterpresse abfiltriert wird, und das Verfahren der Schichtfiltration, bei dem zunächst durch Aufschwemmen auf dem Filter eine Adsorptionsmittelschicht erzeugt wird, durch die das zu reinigende Edukt hindurchgedrückt wird.

Für die erfindungsgemäße Reinigung von EGC durch Adsorption an Aktivkohle werden bevorzugt Festbettverfahren herangezogen. In vorteilhafter Weise werden zwei Adsorptionsbetten vorgesehen, um bei Erschöpfung des einen Adsorptionsbettes auf das andere umschalten und die verbrauchte Aktivkohle regenerieren bzw. ersetzen zu können. Je nach gewähltem Verfahren muß der erfindungsgemäßen Reinigung des EGC durch Adsorption ein Filtrationsschritt nachgeschaltet werden.

Die bei der erfindungsgemäßen Reinigung des EGC durch Adsorption einzustellenden Parameter, wie Temperatur, Druck, Verweilzeit, sind von der Wahl des Adsorptionsverfahrens abhängig und beeinflussen in einer dem Fachmann bekannten Weise das Ergebnis der Reinigung. Beispielsweise muß für die Reinigung des EGC eine Temperatur oberhalb des Schmelzpunktes des EGC, d.h. oberhalb ca. 40°C eingehalten werden. Maximal möglich ist die Siedetemperatur des EGC, also 250°C. Da sich das EGC, insbesondere bei Katalysatorenanwesenheit, bei höherer Temperatur zersetzen kann, empfiehlt es sich bei Wahl einer höheren Temperatur auch höhere Drücke anzuwenden.

Prinzipiell sind als Adsorptionstemperatur also 40 bis 250°C, bevorzugt 40 bis 200°C und besonders bevorzugt 40 bis 160°C einzustellen. Der Druck soll im Bereich 1 bis 200 bar, bevorzugt 1 bis 100 bar, besonders bevorzugt 1 bis 30 bar liegen.

Die erfindungsgemäße Reinigung von EGC durch Behandlung mit Aktivkohle wird bevorzugt unter Ausschuß von Feuchtigkeit durchgeführt.

Unter Aktivkohle im Sinne der vorliegenden Erfindung ist aktivierter Kohlenstoff zu verstehen, der aus unterschiedlichen, Kohlenstoff liefernden Vorprodukten hergestellt werden kann. Die Verfahren zur Überführung in die aktive Form können ebenfalls sehr unterschiedlich sein. Bei solchen Herstellungsverfahren werden Aktivkohlen erhalten, die BET-Oberflächen von 200 bis 3 000 m²/g, bevorzugt 300 bis 2 000 m²/g, besonders bevorzugt 500 bis 1 500 m²/g und Schüttgewichte zwischen 250 und 550 g/l aufweisen. Als Ausgangsmaterialien zur Herstellung aktivierter Kohlen seien beispielsweise genannt: Sägemehl und andere Holzabfalle, Stroh, verschiedene Kohlensorten, wie Bitumen- oder Braunkohle, Nußschalen, Mineralölteere, Lignin, Polysaccharide, Polyacrylnitril, Knochen, Torf. Ferner können auch Koksprodukte aus Braun- und Steinkohlen eingesetzt werden. In bevorzugter Weise seien genannt: Holz, Cellulose, Lignin, Bitumen- oder Braunkohle, Torf oder Steinkohlenkoks.

Die genannten Kohlenstoff liefernden Vorprodukte können durch verschiedene Methoden aktiviert werden, beispielsweise durch chemische Aktivierung mit Phosphorsäure oder Zinkchlorid, durch Gasaktivierung mit Dampf, Sauerstoff oder Nitrose enthaltenden Gase. Solche voraktivierten Vorprodukte werden sodann thermisch, d.h. durch Verkoken in Aktivkohlen für das erfindungsgemäße Verfahren übergeführt. Diese Herstellungsweisen sind dem Fachmann bekannt und sind genau wie die nähere Beschreibung der dabei erhältlichen verschiedenen Sorten von Aktivkohle in der Literatur ausführlich beschrieben (s. Ullmann's Encyclopedia of Industrial Chemistry, 5th, Ed., Vol. A5 (1986), S. 124-140 und die dort zitierte Literatur).

Hinsichtlich der Anwendungsform können in das erfindungsgemäße Verfahren Formkohlen, gebrochene Kohlen und Pulverkohlen eingesetzt werden. Bei Formkohlen, die meist durch Strangpressen aus Pulvern hergestellt werden und dann zylindrische Form aufweisen oder seltender als Pellets vorliegen, kommen meist Durchmesser im Bereich von ein bis einigen mm in Frage. Bei Pulverkohlen muß besonders auf ausreichende Filtrierbarkeit geachtet werden.

Dem Fachmann ist die von der Wahl des Adsorptionsverfahrens abhängige optimale Anwendungsform der Aktivkohle bekannt.

Eine Regenerierung lohnt häufig nicht. Die adsorbierten Salze und farbgebenden Verbindungen können gegebenenfalls mit Wasser, mit Methanol, Methanol/Wasser, mit Glykol oder mit Glykol-Wasser-Gemischen von der Aktivkohle abgewaschen und auf diese Weise eine Regenerierung erreicht werden. Bei kontinuierlicher Arbeitsweise kann die eingesetzte Aktivkohle über lange Zeit im Adsorber verbleiben. Unlösliche organische Ablagerungen können durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 5 Gew.-% Sauerstoff enthaltenden Verdünnungsgasen wie Stickstoff, Kohlenmonoxid oder Kohlendioxid oder durch Kohlendioxid allein bei 200 bis 800°C entfernt werden. Die bevorzugte Regenerierungstemperatur liegt bei 250 bis 700°C, besonders bevorzugt bei 250 bis 600°C.

### Beispiele

Die folgenden Beispiele zeigen die kontinuierliche und die absatzweise Kontaktierung der Aktivkohle mit dem zu reinigenden EGC und ebenfalls die Auswirkung der Aktivkohleadsorption auf den im zu reinigenden EGC gelösten Katalysator.

Die Zusammensetzung der EGC-Katalysatorlösung und des gereinigten EGC wurden gaschromatographisch ermittelt. Als empfindliches Maß für die Farbreinheit von Roh- und Reinprodukt wurde die Farbzahl nach Hazen verwendet, die den beispielsweise auf die polykondensierten Folgeprodukte des Acetaldehyds zurückzuführenden Gelbstich in den EGC-Proben quantifiziert.

Die Katalysatorkonzentrationen in Roh- und Rein-EGC wurden bei Verwendung von anorganischen Salzen mit Hilfe der Atom-Absorptions-Spektroskopie, im Falle des Ammoniumhalogenids als Katalysator durch argentometrische Titration ermittelt.

### Beispiel 1 (Tabelle 1)

In absatzweise ausgeführten Versuchen wurden je 5 Teile EGC-Katalysatorlösung und 1 Teil Aktivkohle (Gew.-Verhältnis) bei 50 bis 60°C unter Rühren miteinander kontaktiert. Nach 30 min, 1, 3 bzw. 24 h wurden Proben des EGC entnommen und nach Abtrennung von Aktivkohleresten die Farbzahl nach Hazen und der Gehalt an Katalysator bestimmt.

Folgende fünf Aktivkohlen wurden geprüft:

| |
|---|
| A: Norit R 2030, A-9421* ( ≈ 2 mm, Stäbchen/ 675 m²/g) |
| B: Norit R2, A-5997* ( ≈ 1,5 mm, Stäbchen/ 1100 m²/g) |
| C: Norit RB1, A-9661* ( < 1 mm, Stäbchen/ 1000 m²/g) |
| D: Sorbonorit 2, A-8008* ( ≈ 1,5 mm, Stäbchen/ 1250 m²/g) |
| E: Chemviron CPG LF ** ( 1- 2 mm, gekörnt/1000 m²/g) |

| |
|---|
| *Handelsprodukt der Fa. Norit |
| ** Handelsprodukt der Fa. Calgon |

Bei den fünf Versuchen kam eine identische EGC-Qualität mit einer Farbzahl von 400, einer Beladung eines Katalysators aus NaBr und ZnBr₂ von 0,22 mol-% NaBr und 0,11 mol-% ZnBr₂ zum Einsatz. Die Qualität dieses Einsatzes-EGC's laut GC-Analyse war mit 99,02 % EGC schon sehr hoch.

Tabelle 1 zeigt das Ergebnis der 5 Versuche 1A bis 1E. Als Maß für die Adsorption des Katalysators an der Aktivkohle wird hier die Konzentration an ZnBr₂ in der gereinigten EGC-Lösung aufgetragen.

### Reinigung von EGC mit verschiedenen Aktivkohlen

Kohlen A bis E; Verhältnis: 1 Teil A-Kohle/5 Teile EGC; 50-60°C

| Verweilzeit (h) | **Farbzahl (Hazen** | | | | | **mol-% ZnBr**_{**2**} **(AAS)** | | | | |
|---|---|---|---|---|---|---|---|---|---|---|
| | A | B | C | D | E | A | B | C | D | E |
| **Edukt** | **400** | | | | | **0,11** | | | | |
| 0,5 | 400 | 250 | 250 | 190 | 80 | 0,108 | 0,106 | 0,100 | 0,103 | 0,092 |
| 1 | 400 | 200 | 200 | 190 | 50 | 0,107 | 0,104 | 0,096 | 0,097 | 0,095 |
| 3 | 375 | 180 | 180 | 170 | 30 | 0,106 | 0,099 | 0,087 | 0,097 | 0,120 |
| 24 | 225 | 100 | 120 | 100 | 20 | 0,093 | 0,089 | 0,074 | 0,091 | 0,080 |

### Folgende Erkenntnisse lassen sich aus Beispiel 1 ableiten:

* Für die erfindungsgemäße Reinigung von EGC durch Adsorption eignen sich verschiedene Aktivkohlen.
* Für die Reinigung des EGC am besten geeignet ist unter den hier betrachteten Kohlen die Aktivkohle Chemviron CPG LF. Schon nach 30 min sinkt die Farbzahl des EGC von 400 auf 80, nach 3 h beträgt sie nur noch 30.
* Alle Aktivkohlen adsorbieren je nach Verweilzeit etwa 10 bis 20 % des Katalysators.

### Beispiel 2

In einem weiteren, absatzweise durchgeführten Versuch wurden 5 Teile EGC-Katalysatorlösung (99,7 % EGC laut GC-Analyse, Farbzahl 35, 1,21 Gew.-% Et₄NBr als Katalysator) und 1 Teil Aktivkohle (Chemviron CPG LF, siehe Beispiel 1) bei 50 bis 60°C unter Rühren miteinander kontaktiert. Nach 30 min wurde eine Probe des EGC entnommen und von Aktivkohleresten befreit. Die Farbzahl nach Hazen betrug dann 10 und der Gehalt an Katalysator sank gegenüber der Ausgangsprobe um 5 %.

### Beispiel 3

Die Adsorption wurde kontinuerlich an der Aktivkohle Chemviron CPG LF in einer auf 65°C temperierten Säule (2 cm ⌀) durchgeführt, die im geflutetem Zustand von oben nach unten mit konstantem Volumenstrom mit dem zu reinigenden EGC durchströmt wurde. Jeweils im Abstand einiger Stunden wurden momentane Proben des Ablaufs gezogen und analysiert.

Das eingesetzte EGC hatte eine Farbzahl von 80 (Betthöhe 16 cm, 50 ml Bettvolumen, Schüttdichte 0,46 g/ml_{Bett}, Trocknung der Aktivkohle vor Versuchsbeginn 36 h bei 130°C Stickstoff; 10 min Verweilzeit).

7,1 l des EGC mit einer Farbzahl von 80 wurden in diesem Versuch an 50 ml Chemviron CPG LF auf eine Farbzahl von maximal 55 gereinigt (142-faches Volumen des Adsorptionsbettes). Zu Beginn des Versuches erhielt man sogar 2 l Eluat mit einer Farbzahl von nur 35 bis 40.

## Patentansprüche

1. Verfahren zur kontinuierlichen oder diskontinuierlichen Reinigung von Ethylenglykolcarbonat (EGC), welches Verunreinigungen aus dem Herstellungs- bzw. Aufarbeitungsprozeß des EGC enthält, dadurch gekennzeichnet, daß das verunreinigte EGC bei 40-250°C und bei 1-200 bar einer Behandlung mit Aktivkohle mit einer BET-Oberfläche von 200-3000 m² unterworfen wird.

2. Verfahren nach Anspruch 1, dadurch gekennzeichnet, daß es sich bei dem zu reinigenden EGC um den zur Vermeidung einer Akkumulation von Verunreinigungen aus dem EGC-Reakfions- und Aufarbeitungskreislauf herausgeführten Ausschleusestrom und/oder um einen Ausschleusestrom aus dem Rückstand der EGC-Aufarbeitung handelt.

3. Verfahren nach Anspruch 2, dadurch gekennzeichnet, daß der Ausschleusestrom neben Verunreinigungen auch den Katalysator aus dem EGC-Herstellungsprozeß enthält und die Behandlung mit Aktivkohle gleichzeitig die teilweise Entfernung des Katalysators bewirkt.

4. Verfahren nach Anspruch 3, dadurch gekennzeichnet, daß der Ausschleusestrom nur teilweise einer Aktivkohlereinigung zugeführt wird und/oder daß bei dieser Reinigung nur eine teilweise Entfernung der Nebenkomponenten herbeigeführt wird.

5. Verfahren nach Anspruch 2, dadurch gekennzeichnet, daß der durch Behandlung mit Aktivkohle gereinigte Ausschleusestrom in den Reaktions- und Aufarbeitungskreislauf im EGC-Herstellungsprozeß zurückgeführt wird.

6. Verfahren nach Anspruch 1, dadurch gekennzeichnet, daß die Aktivkohle in einem Festbett angeordnet ist.

7. Verfahren nach Anspruch 1, dadurch gekennzeichnet, daß nach dem Adsorptionsschritt eine Filtration durchgeführt wird.

8. Verfahren nach Anspruch 1, dadurch gekennzeichnet, daß die Reinigung unter Ausschluß von Feuchtigkeit stattfindet.

9. Verfahren nach Anspruch 1, dadurch gekennzeichnet, daß eine Aktivkohle mit einem Schüttgewicht zwischen 250 und 550 g/l eingesetzt wird.

10. Verfahren nach Anspruch 1, dadurch gekennzeichnet, daß eine Aktivkohle eingesetzt wird, die aus Sägemehl und anderen Holzabfällem, Stroh, Kohlensorten, Nußschalen, Mineralölteeren, Lignin, Polysacchariden, Polyacrylnitril, Knochen, Torf oder Koksprodukten aus Braun- oder Steinkohlen, bevorzugt aus Holz, Cellulose, Lignin, Bitumen- oder Braunkohle, Torf oder Steinkohlenkoks hergestellt wurde.

## Claims

1. Process for the continuous or batchwise purification of ethylene glycol carbonate (EGC) containing impurities from the production or work-up process of the EGC, which is characterized in that the contaminated EGC is subjected at 40-250°C and at 1-200 bar to a treatment with activated carbon having a BET surface area of 200-3,000 m².

2. Process according to Claim 1, characterized in that the EGC to be purified is a bleed stream taken from the EGC reaction and work-up circuit to avoid accumulation of impurities and/or is a bleed stream from the residue of the EGC work-up.

3. Process according to Claim 2, characterized in that the bleed stream contains, besides impurities, the catalyst from the EGC production process and the treatment with activated carbon at the same time effects the partial removal of the catalyst.

4. Process according to Claim 3, characterized in that the bleed stream is fed only partially to activated carbon purification and/or in that in this purification only partial removal of the secondary components is brought about.

5. Process according to Claim 2, characterized in that the bleed stream purified by treatment with activated carbon is recycled into the reaction and workup circuit in the EGC production process.

6. Process according to Claim 1, characterized in that the activated carbon is arranged in a fixed bed.

7. Process according to Claim 1, characterized in that filtration is carried out after the adsorption step.

8. Process according to Claim 1, characterized in that the purification takes place with exclusion of moisture.

9. Process according to Claim 1, characterized in that the activated carbon used has a bulk density between 250 and 550 g/l.

10. Process according to Claim 1, characterized in that the activated carbon used has been prepared from sawdust and other wood wastes, straw, types of coal, nut shells, mineral oil tars, lignin, polysaccharides, polyacrylonitrile, bones, peat or coke products from brown or black coals, preferably from wood, cellulose, lignin, bituminous or brown coal, peat or black coal coke.

## Revendications

1. Procédé pour la purification en continu ou en discontinu d'éthylèneglycolcarbonate (EGC) qui contient des impuretés provenant du processus de préparation, respectivement du processus de retraitement du EGC, caractérisé en ce qu'on soumet le EGC pollué, à une température de 40-250°C et sous une pression de 1-200 bar, à un traitement avec du charbon actif possédant une surface BET de 200-3000 m²/g.

2. Procédé selon la revendication 1, caractérisé en ce qu'il s'agit, en ce qui concerne le EGC à purifier, du courant d'éclusage au-dehors guidé à l'extérieur du circuit de réaction et de retraitement du EGC pour éviter une accumulation d'impuretés et/ou d'un courant d'éclusage de sortie constitué par le résidu du retraitement du EGC.

3. Procédé selon la revendication 2, caractérisé en ce que le courant d'éclusage au-dehors contient, outre des impuretés, également le catalyseur provenant du procédé de préparation du EGC, et le traitement avec du charbon actif a pour effet simultané d'éliminer en partie le catalyseur.

4. Procédé selon la revendication 3, caractérisé en ce que le courant d'éclusage au-dehors n'est acheminé que partiellement à une purification à l'aide de charbon actif et/ou en ce qu'au cours de cette purification, on n'obtient qu'une élimination partielle des sous-composants.

5. Procédé selon la revendication 2, caractérisé en ce que le courant d'éclusage au-dehors purifié lors du traitement avec du charbon actif est renvoyé dans le circuit de réaction et de retraitement dans le procédé de préparation du EGC.

6. Procédé selon la revendication 1, caractérisé en ce que le charbon actif est disposé dans un lit fixe.

7. Procédé selon la revendication 1, caractérisé en ce qu'on procède à une filtration après l'étape d'adsorption.

8. Procédé selon la revendication 1, caractérisé en ce que la purification a lieu en l'absence d'humidité.

9. Procédé selon la revendication 1, caractérisé en ce qu'on met en oeuvre un charbon actif possédant une masse volumique apparente entre 250 et 550 g/l.

10. Procédé selon la revendication 1, caractérisé en ce qu'on met en oeuvre un charbon actif qui a été préparé à partir de sciure de bois et d'autres déchets de bois, de paille, de sortes de charbon, d'écorces de noix, de goudrons d'huiles minérales, de lignine, de polysaccharides, de polyacrylonitrile, d'os, de tourbe ou de produits de coke constitués de lignite ou de houilles, de préférence de bois, de cellulose, de lignine, de charbon bitumineux ou de lignite, de tourbe ou encore de coke de houille.
